# EUROPÄISCHE PATENTSCHRIFT

(11) **EP 1 918 208 B1**
(45) Veröffentlichungstag und Bekanntmachung des Hinweises auf die Patenterteilung: **17.10.2012**
(21) Anmeldenummer: 07021174.3
(22) Anmeldetag: 30.10.2007
(51) Int. Cl.: B65B 55/08, A61L 2/10

(54) **Packstoff-Entkeimungsanlage**
Packaging material sterilisation facility
Installation de désinfection de matériaux d'emballage

(30) Priorität: 30.10.2006 DE 102006051738
(43) Veröffentlichungstag der Anmeldung: 07.05.2008
(73) Patentinhaber: Heraeus Noblelight GmbH, 63450 Hanau (DE)
(72) Erfinder: Brieden, Karl-Wilhelm, 63579 Freigericht (DE); Greif, Stefan, 36039 Fulda (DE); Lowitzki, Jürgen, 55442 Daxweiler (DE)
(74) Vertreter: Kühn, Hans-Christian

(56) Entgegenhaltungen:
- EP-A- 0 065 380
- EP-A- 0 277 505
- EP-A- 1 020 363
- EP-A- 1 103 300
- EP-A- 1 186 913
- EP-A- 1 225 614
- WO-A-96/09775

## Beschreibung

Die vorliegende Erfindung betrifft eine Packstoff-Entkeimungseinrichtung für eine Füll- und Verschließanlage, insbesondere mit einer Taktung unter 5 Sekunden, deren Verwendung zur Entkeimung, sowie Verfahren zur Entkeimung.

Es sind Packstoff-Entkeimungseinrichtungen bekannt, die mittels Gasentladungsröhren UV-Strahlung emittieren.

Gemäß EP 0 591 001 werden UV-Entladungsröhren einseitig mit metallischen Reflektoren bestückt.

Es lassen sich mehrere mit Reflektoren hinterlegte UV-Strahler parallel nebeneinander anordnen, so dass ein homogenes Bestrahlungsfeld erzeugt wird. Dies führt jedoch bei der Entkeimung von wärmeempfindlichen Substanzen wie beispielsweise Joghurtbechem zu einem unerwünschten Aufheizen des Bestrahlungsgutes sowie einer Ineffizienz in der Ausnutzung der UV-Strahlung, da das Stückgut die bestrahlte Fläche nicht vollständig bedeckt. Die dabei ungenutzte UV-Strahlung hat den weiteren negativen Begleiteffekt, dass sie zur Beschädigung der Außenwände des bestrahlten Gutes oder Alterung der bestrahlten Anlagenteile, beispielsweise des Förderbandes, führt. Insbesondere werden bei Stillstand eines Förderbandes diese Nachteile sich noch gravierender auswirken, so dass sogar Brandgefahr durch das Bestrahlungsgut entsteht.

DE 10 2004 054 662 betrifft die Innenbehandlung von Hohlprofilen.

EP 1 133 705 offenbart einen dielektrischen Reflektor mit selektiver Durchlässigkeit, der einen dielektrischen Stapel aus optischen Wiederholeinheiten mit mindestens zwei verschiedenen Materialien mit unterschiedlichen Brechungsindizes aufweist, wobei der Stapel einen Grenzwinkel aufweist, bei dem sich im Stapel ausbreitendes Licht an einer Grenzfläche des Stapels mit Luft eine innere Totalreflexion erfährt.

EP 0 277 505 offenbart die Sterilisation von Flaschen mit einer Hoch- oder Höchstdruck-Quecksilberlampe und einem mehrlagig beschichteten Reflektor. Vorzugsweise weist der Reflektor eine Aluminiumbeschichtung auf. Zur selektiven Reflektion von UV-Strahlung im Bereich von 200 bis 300 nm, z. B. 254 nm, weist die Reflektoroberfläche eine mehrlagige Beschichtung auf. Die Desinfektion erfolgt über optische Fasern, auf die die Strahlung fokussiert wird.

Die Aufgabe der vorliegenden Erfindung besteht darin, die Entkeimung bezüglich der eingesetzten UV-Strahlung effizienter zu gestalten und die Strahlungsbelastung des Bestrahlungsgutes auf die minimale zur Entkeimung notwendige Dosis zu beschränken.

Erfindungsgemäß wurde erkannt, dass eine vollflächige, kalte und besonders intensive, sehr kurze, desinfizierende Strahlung für die Massenproduktion, insbesondere Füll- und Verschließanlagen, besonders geeignet ist.

Die Lösung der Aufgabe erfolgt mit den unabhängigen Ansprüchen. In den abhängigen Ansprüchen sind bevorzugte Ausführungsformen beschrieben.

Erfindungsgemäß enthält eine Packstoff-Entkeimungseinrichtung in einer Füll- und Verschließanlage wenigstens einen UV-Hoch- (medium) oder Höchstdruck-Entladungsstrahler (high pressure lamp) und einen für IR-Strahlung durchlässigen Glasreflektor, der eine UV-C reflektierende Beschichtung aufweist, wobei der Entladungsbogen des UV-Strahlers in der Symmetrieachse des Reflektors angeordnet wird.

Damit die desinfizierende Strahlung möglichst intensiv und gleichmäßig auf die Innenwand oder Innenwände achsensymmetrischer Hohlkörper verteilt wird, ist es vorteilhaft, einen Lichtbogen mit hoher Leistung in der Symmetrieachse des Reflektors anzuordnen.

Mit dem in der Symmetrieachse des Reflektors angeordneten Lichtbogen verbessert sich die Tiefenwirkung bei achsensymmetrischem Packmaterial.

Die erfindungsgemäße Packstoff-Entkeimungseinrichtung eignet sich deshalb besonders zur Entkeimung achsensymmetrischer Innenflächen einseitig offener Hohlkörper, beispielsweise von Bechern oder Flaschen, insbesondere Kunststoffbechern für Joghurt und Desserts.

Auf diese Weise wird praktisch die gesamte UV-C-Strahlung auf die zu entkeimende Oberfläche insbesondere innerhalb eines Behälters geleitet, wobei ein Großteil der IR-Strahlung durch den Reflektor entweicht, der aus diesem Grunde aus Glas beschaffen ist und lediglich eine UV-C reflektierende Schicht aufweist. Die Projektion auf die Gehäuse-Innenfläche erlaubt eine schonende UV-Bestrahlung mit Strahlern geringer elektrischer Anschlussleistung. Die geringe Strahlerleistung und der für IR-Strahlung transparente Reflektor ermöglichen eine geringe Wärmebelastung des Packstoffes. Damit einhergehend werden kompaktere Entkeimungseinrichtungen bereitgestellt, die wiederum für kleinere Maschinen besonders geeignet sind.

Erfindungsgemäß wird auch mit einer höheren UV-C-Dosis bei sonst geringerer Bestrahlung eine kürzere Bestrahlungszeit ermöglicht. Damit einhergehend lässt sich die Taktzeit beim diskontinuierlichen Betrieb herabsetzen, beispielsweise auf 1 bis 2 Sekunden.

In bevorzugten Ausführungen
■ weist der UV-Hoch- oder Höchstdrurk-Entladungsstrahler eine elektrische Anschlussleistung zwischen 50 und 500 W auf, insbesondere 100 bis 400 W;
■ beträgt die Länge des Entladungsbogens des UV-Strahlers zwischen 2 und 50 mm, insbesondere 5 bis 20 mm;
■ ist die UV reflektierende Beschichtung des Glasreflektors eine Interferenzbeschichtung für UV-GStrahlung;
■ weist die UV-C reflektierende Beschichtung des Glasreflektors HfO₂-Schichten auf;
■ ist der Reflektor für VIS-Strahlung durchlässig,
■ ist der Reflektor für UV-A-Strahlung durchlässig;
■ ist der Reflektor für UV-B-Strahlung durchlässig.

Insbesondere beträgt die VIS- und IR-Durchlässigkeit 50 bis 80 % bei einem Auftragswinkel bis 52° (x π + 180° [rad]).

Als UV reflektierende Schicht hat sich eine UV-C reflektierende Mehrschichten-Interferenzbeschichtung bewährt, beispielsweise aus HfO₂- und SiO₂-Wechselschichten oder Ta₂O₅- und SiO₂-Wechselschichten.

Besonders bewährt haben sich segmentierte Reflektoren. Ihr Vorteil besteht in einem geringen Justieraufwand gegenüber Reflektoren mit kontinuierlicher Reflektoroberfläche.

Es hat sich bewährt, in einer Entkeimungsanlage mehrere Strahler mit jeweils einem Reflektor nebeneinander anzuordnen, um die gleichzeitige Bestrahlung mehrerer Bestrahlungsgut-Gegenstände zu ermöglichen.

Einhergehend mit der Anordnung des Bogens in der Symmetrieachse des Reflektors wird die UV-C-Strahlung im Wesentlichen indirekt über den Reflektor der zu bestrahlenden Packmitteloberfläche zugeführt. Dies wiederum bewirkt, dass das erfindungsgemäße Verfahren zur Entkeimung von Packstoff, insbesondere von einseitig offenen Hohlkörpern, sehr effizient ist. Erfindungsgemäß wird hierzu die UV-C-Strahlung eines UV-Strahlers mit einem selektiven UV-C-Reflektor auf die Innenseiten des Packstoffes reflektiert. Ganz besonders bevorzugt werden dabei durch UV-Strahlung initiierte OH-Radikale gebildet, insbesondere aus Peroxiden wie H₂O₂ oder Peroxyessigsäure. Hierzu reicht bereits eine 3%ige WasserstoffperoxidLösung, für die relativ geringe Sicherheitsmaßnahmen erforderlich sind. Die Behandlung der zu entkeimenden Oberfläche mit OH-Radikalbildnern erfolgt vorzugsweise durch Besprühen vor der Bestrahlung.

Im Folgenden wird die Erfindung anhand von Beispielen mit Bezug auf die Abbildungen verdeutlicht.

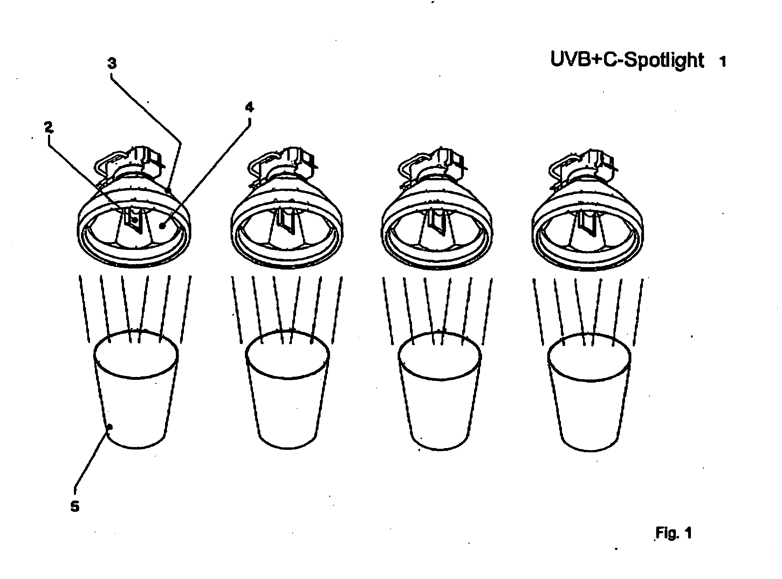

Figur 1 zeigt vier nebeneinander angeordnete UV-C-Spotlights 1 aus je einem Hg-Mitteldruckstrahler 2, die über je einem Joghurtbecher 5 angeordnet sind. Die Mitteldruckstrahler 2 sind jeweils auf der Achse eines für IR-Strahlung durchlässigen Glasreflektors 3 angeordnet. Der Glasreflektor 3 weist eine reflektierende Beschichtung 4 für UV-Strahlung auf. Eine derartige Anordnung eignet sich besonders für den diskontinuierlichen Betrieb (stop and go).

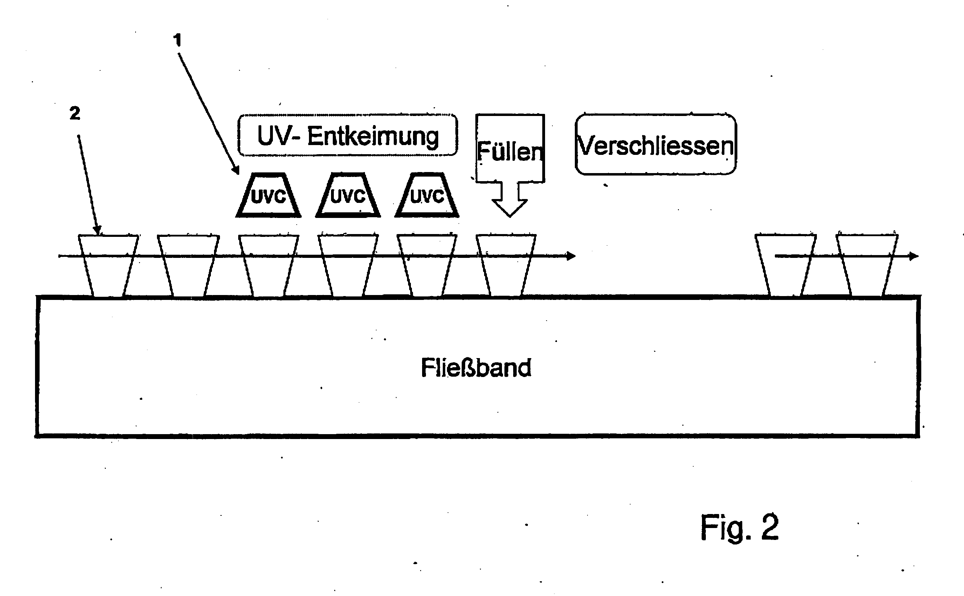

Figur 2 zeigt eine Füll- und Verschließanlage im schematischen Aufbau. Auf einem Fließband werden hier Kunststoffbecher 5 von links nach rechts transportiert und erfahren mit UV-C-Spotlights 1 eine UV-Entkeimung, bevor sie befüllt werden, beispielsweise mit Joghurt.

Nach dem Füllen erfolgen ein Verschließen und der Abtransport der fertigen Ware, wie beispielsweise verpackter Joghurt.

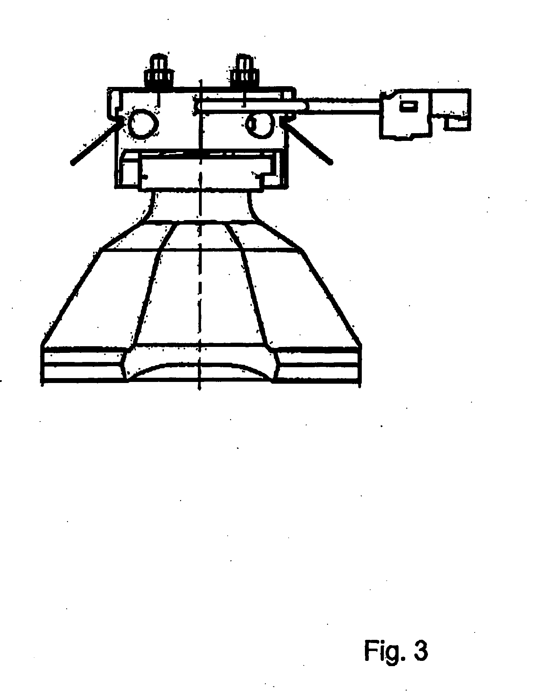

Figur 3 zeigt einen segmentierten Reflektor, dessen Glasgrundkörper und Bajonettbefestigung identisch sind mit dem MH lamp modul 250 Watt von Heraeus. Der Unterschied zum bekannten Strahler besteht in der innenseitigen UV-C-Beschichtung. Der Bajonettverschluss ermöglicht eine leichte Reflektormontage und das Auswechseln von Strahlern unter Weiterverwendung des Reflektors.

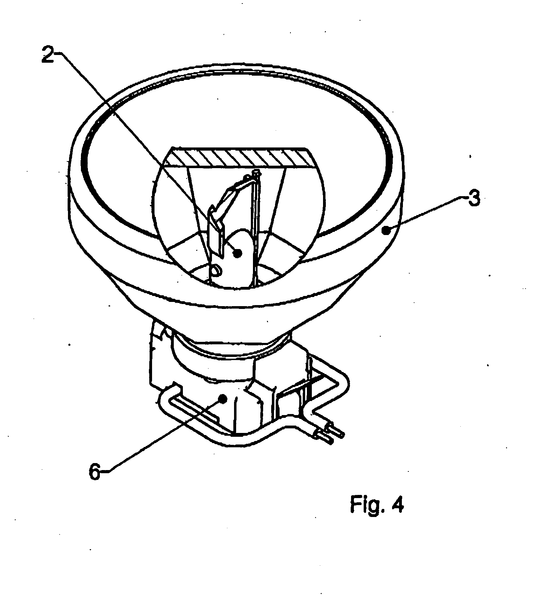

Figur 4 zeigt eine Vergrößerung des UV-C-Spotlights 1 aus Figur 1 mit einem Bajonettverschluss 6.

## Patentansprüche

1. Packstoff-Entkeimungseinrichtung einer Füll- und Verschließanlage, enthaltend wenigstens einen UV-Hoch- oder Höchstdruck-Entladungsstrahler und einen für IR-Strahlung durchlässigen Glasreflektor, der eine UV-C reflektierende Beschichtung aufweist, wobei der Entladungsbogen des UV-Strahlers in der Symmetrieachse des Reflektors angeordnet ist, und wobei die Entkelmungseinrichtung einen Reflektor zur vollflächigen Projektion von UV-C-Strahlung auf eine radialsymmetrische Fläche oder eine zylindrische, kegelförmige, konische oder halbkugelförmige Innenfläche eines Hohlkörpers oder Kombinationen davon aufweist.

2. Packstoff-Entkeimungseinrichtung nach Anspruch 1, **dadurch gekennzeichnet, dass** der UV-Hoch- oder Höchstdruck-Entladungsstrahler eine elektrische Anschlussleistung von 100 bis 400 W aufweist.

3. Packstoff-Entkeimungseinrichtung nach Anspruch 1 oder 2, **dadurch gekennzeichnet, dass** der Entladungsbogen des UV-Strahlers 0,5 bis 2 cm lang ist.

4. Packstoff-Entkeimungseinrichtung nach einem der Ansprüche 1 bis 3, **dadurch gekennzeichnet, dass** die UV reflektierende Beschichtung des Glasreflektors eine Interferenzbeschichtung für UV-C-Strahtung ist.

5. Packstoff-Entkeimungseinrichtung nach einem der Ansprüche 1 bis 4, **dadurch gekennzeichnet, dass** die UV-C reflektierende Beschichtung des Glasreflektors HfO₂- oder Ta₂O₅-Schichten aufweist.

6. Packstoff-Entkeimungseinrichtung nach einem der Ansprüche 1 bis 5, **dadurch gekennzeichnet, dass** der Reflektor für VIS-Strahlung durchlässig ist

7. Packstoff-Entkeimungseinrichtung nach einem der Ansprüche 1 bis 6, **dadurch gekennzeichnet, dass** die Füll- und Verschließanlage eine Taktung unterhalb 5 Sekunden aufweist.

8. Packstoff-Entkeimungseinrichtung nach einem der Ansprüche 1 bis 7, **dadurch gekennzeichnet, dass** die UV-C-Strahlung nicht zu einem Brennpunkt fokussiert ist.

9. Verfahren zur Entkeimung von Packstoffen mittels einer Entkeimungseinrichtung gemäß einem des Ansprüch 1 bis 8, **dadurch gekennzeichnet, dass** UV-C-Strahlung eines UV-Strahlers mit einem selektiven UV-C-Reflektor auf zu entkeimende Oberflächen gerichtet wird.

10. Verfahren zur Entkeimung von Packstoffen mittels UV-Strahlung, **dadurch gekennzeichnet, dass** UV-C-Strahlung eines UV-Strahlers mit einem selektiven UV-C-Reflektor auf die Innenseiten einseitig offener Hohlkörper reflektiert wird.

11. Verfahren nach Anspruch 9 oder 10, **dadurch gekennzeichnet, dass** der Packstoff mit einem OH-Radikalbildner behandelt wird, der bei der Bestrahlung mit UV-C OH-Radikale bildet.

12. Verfahren nach einem der Ansprüche 9 bis 11 zur Entkeimung von Packstoffen, die als Hohlkörper ausgebildet sind, **dadurch gekennzeichnet, dass** in einer Füll- und Verschließanlage mit einem UV-Hoch- oder Höchstdruck-Entladungsstrahler eine kalte Desinfektion der gesamten Innenfläche des Hohlkörpers mittels UV-C selektivem Reflektor erfolgt.

13. Verfahren nach einem der Ansprüche 9 bis 11 zur Entkeimung kreisrunder Flächen zum Verschluss von Hohlkörpern in einer Packstoff-Entkeimungseinrichtung einer Füll- und Verschließanlage, **dadurch gekennzeichnet, dass** mit einem UV-Hoch- oder Höchstdruck-Entladungsstrahler die für die Innenseite vorgesehene Fläche mit einer kalten UV-C-Strahlung desinfiziert wird.

14. Verwendung einer Packstoff-Entkeimungseinrichtung nach einem der Ansprüche 1 bis 6, **dadurch gekennzeichnet, dass** eine kalte desinfizierende Strahlung vollflächig auf die Innenseite eines Hohlkörpers gestrahlt wird.

15. Verwendung nach Anspruch 12 oder 14, **dadurch gekennzeichnet, dass** die Bestrahlung innerhalb von 5 Sekunden erfolgt.

## Claims

1. Packaging materials disinfection facility of a filling and sealing plant, containing at least one UV high-pressure or highest-pressure discharge radiator and an IR radiation-translucent glass reflector that comprises a UV-C-reflecting coating, whereby the discharge arc of the UV radiator is arranged in the symmetry axis of the reflector, and whereby the disinfection facility comprises a reflector for full-surface projection of UV-C radiation onto a radially-symmetrical surface or a cylindrical, cone-shaped, conical or semispherical internal surface of a hollow body or combinations thereof.

2. Packaging materials disinfection facility according to claim 1, **characterised in that** the UV high-pressure or highest-pressure discharge radiator has an electrical connected load of 100 to 400 W.

3. Packaging materials disinfection facility according to claim 1 or 2, **characterised in that** the discharge arc of the UV radiator is 0.5 to 2 cm in length.

4. Packaging materials disinfection facility according to any one of the claims 1 to 3, **characterised in that** the UV-reflecting coating of the glass reflector is an interference coating for UV-C radiation.

5. Packaging materials disinfection facility according to any one of the claims 1 to 4, **characterised in that** the UV-reflecting coating of the glass reflector comprises layers of HfO₂ or Ta₂O₅.

6. Packaging materials disinfection facility according to any one of the claims 1 to 5, **characterised in that** the reflector is translucent for VIS radiation.

7. Packaging materials disinfection facility according to any one of the claims 1 to 6, **characterised in that** the filling and sealing plant has a timing of less than 5 seconds.

8. Packaging materials disinfection facility according to any one of the claims 1 to 7, **characterised in that** the UV-C radiation is not being focussed to a focal point.

9. Method for disinfection of packaging materials by means of a disinfection facility according to any one of the claims 1 to 8, **characterised in that** UV-C radiation of a UV radiator is directed onto surfaces to be disinfected by means of a selective UV-C reflector.

10. Method for disinfection of packaging materials by means of UV radiation, **characterised in that** UV-C radiation of a UV radiator is reflected onto the internal surfaces of hollow bodies that are open on one side by means of a selective UV-C reflector.

11. Method according to claim 9 or 10, **characterised in that** the packaging material is treated with an OH radical-forming agent that forms OH radicals upon being irradiated with UV-C.

12. Method according to any one of the claims 9 to 11 for disinfection of packaging materials provided as hollow bodies, **characterised in that** a cold disinfection of the entire internal surface of the hollow body is carried out in a filling and sealing plant with a UV high-pressure or highest-pressure discharge radiator by means of an UV-C-selective reflector.

13. Method according to any one of the claims 9 to 11 for disinfection of circular surfaces for sealing hollow bodies in a packaging material disinfection facility of a filling and sealing plant, **characterised in that** the surface designed to be the internal surface is disinfected through cold UV-C radiation using a UV high-pressure or highest-pressure discharge radiator.

14. Use of a packaging material disinfection facility according to any one of the claims 1 to 6, **characterised in that** a cold disinfecting radiation is radiated in full-surface manner onto the internal surface of a hollow body.

15. Use according to claim 12 or 14, **characterised in that** the radiation is effected within 5 seconds.

## Revendications

1. Dispositif de stérilisation de matériau d'emballage d'une installation de remplissage et de capsulage contenant au moins une lampe à décharge à pression élevée ou maximale à UV et un réflecteur de verre transparent pour le rayonnement infrarouge qui présente un revêtement réfléchissant les UV-C, dans lequel l'arc de décharge de la lampe UV est disposé dans l'axe de symétrie du réflecteur et dans lequel le dispositif de stérilisation présente un réflecteur pour la projection sur toute la surface de rayonnement UV-C sur une surface à symétrie radiale ou une surface interne cylindrique, conique ou hémisphérique d'un corps creux ou de combinaisons de celles-ci.

2. Dispositif de stérilisation de matériau d'emballage selon la revendication 1, **caractérisé en ce que** la lampe à décharge à pression élevée ou maximale à UV présente une puissance de raccord électrique de 100 à 400 W.

3. Dispositif de stérilisation de matériau d'emballage selon la revendication 1 ou 2, **caractérisé en ce que** l'arc de décharge de la lampe UV est de 0,5 à 2 cm de long.

4. Dispositif de stérilisation de matériau d'emballage selon l'une quelconque des revendications 1 à 3, **caractérisé en ce que** le revêtement réfléchissant les UV du réflecteur de verre est un revêtement à interférence pour rayonnement UV-C.

5. Dispositif de stérilisation de matériau d'emballage selon l'une quelconque des revendications 1 à 4, **caractérisé en ce que** le revêtement réfléchissant les UV-C du réflecteur de verre présente des couches de HfO₂ ou Ta₂O₅.

6. Dispositif de stérilisation de matériau d'emballage selon l'une quelconque des revendications 1 à 5, **caractérisé en ce que** le réflecteur est transparent pour le rayonnement VIS.

7. Dispositif de stérilisation de matériau d'emballage selon l'une quelconque des revendications 1 à 6, **caractérisé en ce que** l'installation de remplissage et de capsulage présente une cadence inférieure à 5 secondes.

8. Dispositif de stérilisation de matériau d'emballage selon l'une quelconque des revendications 1 à 7, **caractérisé en ce que** le rayonnement UV-C n'est pas concentré vers un foyer.

9. Procédé de stérilisation de matériaux d'emballage au moyen d'un dispositif de stérilisation selon l'une quelconque des revendications 1 à 8, **caractérisé en ce que** le rayonnement UV-C d'une lampe UV est orienté avec un réflecteur UV-C sélectif vers des surfaces à stériliser.

10. Procédé de stérilisation de matériaux d'emballage au moyen de rayonnement UV, **caractérisé en ce que** le rayonnement UV-C d'une lampe UV est réfléchi avec un réflecteur UV-C sélectif sur les côtés internes de corps creux ouverts d'un côté.

11. Procédé selon la revendication 9 ou 10, **caractérisé en ce que** le matériau d'emballage est traité avec un agent de formation de radicaux OH qui forme des radicaux OH lors de l'irradiation avec des UV-C.

12. Procédé selon l'une quelconque des revendications 9 à 11 pour la stérilisation de matériaux d'emballage qui sont réalisés en tant que corps creux, **caractérisé en ce qu'**une désinfection froide de l'ensemble de la surface interne du corps creux s'effectue au moyen d'un réflecteur sélectif UV-C dans une installation de remplissage et de capsulage avec une lampe à décharge à pression élevée ou maximale à UV.

13. Procédé selon l'une quelconque des revendications 9 à 11 pour la stérilisation de surfaces circulaires pour la fermeture de corps creux dans un dispositif de stérilisation de matériau d'emballage d'une installation de remplissage et de capsulage, **caractérisé en ce que** la surface prévue pour le côté interne est désinfectée avec un rayonnement UV-C froid avec une lampe à décharge à pression élevée ou maximale à UV.

14. Utilisation d'un dispositif de stérilisation de matériau d'emballage selon l'une quelconque des revendications 1 à 6, **caractérisée en ce qu'**un rayonnement désinfectant froid est irradié sur toute la surface vers le côté interne d'un corps creux.

15. Utilisation selon la revendication 12 ou 14, **caractérisée en ce que** l'irradiation s'effectue en l'espace de 5 secondes.
